# EUROPEAN PATENT APPLICATION

(11) **EP 4 647 506 A1**
(43) Date of publication of application: **12.11.2025**
(21) Application number: 24738465.4
(22) Date of filing: 03.01.2024
(51) Int. Cl.: C12Q 1/6806, C12N 15/10

(54) **KIT AND NUCLEIC ACID EXTRACTION METHOD**

(30) Priority: 04.01.2023 CN 202310007104
(71) Applicant: GeneMind Biosciences Company Limited, Shenzhen, Guangdong 518023 (CN)
(72) Inventor: JU, Mingxia, Shenzhen, Guangdong 518023 (CN); KANG, Huanhuan, Shenzhen, Guangdong 518023 (CN); GAO, Yifan, Shenzhen, Guangdong 518023 (CN); ZHOU, Letian, Shenzhen, Guangdong 518023 (CN); ZENG, Lidong, Shenzhen, Guangdong 518023 (CN); ZHANG, Guoliang, Shenzhen, Guangdong 518023 (CN); BI, Jing, Shenzhen, Guangdong 518023 (CN)
(74) Representative: Schiweck Weinzierl Koch Patentanwälte Partnerschaft mbB
(86) International application number: PCT/CN2024/070244
(87) International publication number: WO 2024/146532

(57) **Abstract**

Disclosed is a reagent kit and a nucleic acid extraction method. The kit is used for nucleic acid sample extraction. The kit comprises a hydrogen peroxide solution, and the molarity of the hydrogen peroxide solution is 0.1-2 mol/L. For certain nucleic acid samples, microbial cells included therein are wrapped by substances such as mucus or mucin and are difficult to release. After the hydrogen peroxide solution is added, substances in the microbial cells react with hydrogen peroxide to generate gas, and generated bubbles can destroy mucus or block-shaped samples, causing the samples to be completely liquefied, so as to release the microbial cells wrapped by the substances such as mucus or mucin, so that subsequent nucleic acid extraction steps can be smoothly carried out.

## Description

### TECHNICAL FIELD

The present application relates to the field of biotechnology, in particular to a reagent kit and a nucleic acid extraction method.

### BACKGROUND

Whole genome sequencing (WGS) can be used in studying the whole genome of *mycobacterium tuberculosis* (Mtb), and the used samples in the research are mostly sputum samples, which have the properties of high viscosity, high mucin content, difficulties in liquefaction, easiness in agglomeration, etc. The nucleic acid detection using sputum samples needs at least 3 processing procedures conventionally: sputum liquefaction, cell lysis, and nucleic acid extraction.

The conventional sputum liquefaction processing procedures mainly have the following defects: (1) The RNAs in the samples are exceptionally prone to RNase catalytic degradation, as no strong denaturation agents are added to meet the requirement of cell integrity in the independent sputum liquefaction procedure, thereby causing nucleic acid loss; (2) the conventional sputum liquefaction commonly requires the use of sulfydryl reagents and a reagent addition volume of 5-10 times of the sputum volume, giving rise to a relatively large final sample volume and a strong likelihood of nucleic acid degradation and loss; and (3) the processing is relatively complicated and time-consuming, where the processing methods used include the sodium hydroxide method, the DTT (dithiothreitol) method, and the protease method. The sodium hydroxide method is the most common sputum liquefaction method and is relatively simple: the sputum is liquefied using the sodium hydroxide solution with a certain concentration as the principal component at 60-80 °C (or under the room temperature condition), and the method is prone to give rise to nucleic acid loss when used in nucleic acid detection because of the strong alkaline environment involved. The DTT (dithiothreitol) method is based on the ability of DTT (dithiothreitol) containing sulfydryl (-SH) to break down mucin that serves as a main ingredient responsible for sputum viscosity. In the process, the PBS buffer solution (phosphate buffered saline) is added to provide physiologic buffering, and reagents, e.g., ethanol and the like, are usually added to fix cells. The use of DTT (dithiothreitol) in the method brings relatively high cost, and the method is prone to give rise to RNA loss and is not suitable for processing a large amount of clinical sputum samples. The protease method is based on the principle of protease degradation of sputum mucin, and the method needs long time consumption in the enzymolysis reaction and a high cost of protease, and has a low efficiency of mucin digestion.

### SUMMARY

In order to reduce nucleic acid loss during the nucleic acid extraction process using viscous samples, such as sputum samples, saliva samples, or pus samples, and to improve the efficiency and yield of nucleic acid extraction, the present application provides a reagent kit and a nucleic acid extraction method.

In a first aspect, the present application provides a reagent kit used in nucleic acid sample extraction, comprising a hydrogen peroxide solution having a molarity of 0.1-2 mol/L. For certain nucleic acid samples, microbial cells included therein are wrapped by substances such as mucus or mucin and are difficult to release. After the hydrogen peroxide solution is added, substances in the microbial cells react with hydrogen peroxide to generate gas, and generated bubbles can destroy mucus or block-shaped samples, causing the samples to be completely liquefied, so as to release the microbial cells wrapped by the substances such as mucus or mucin, so that subsequent nucleic acid extraction steps can be smoothly carried out. In addition, the reaction with hydrogen peroxide solution is relatively mild and substantially brings no destruction to cell integrity. This method is capable of reducing nucleic acid loss and improving nucleic acid yield, compared with those using substances such as strong alkali and the like for liquefaction.

In an example, the hydrogen peroxide solution has a molarity of 0.3-1 mol/L.

In an example, the reagent kit further comprises a lysis solution.

In an example, the lysis solution comprises a first surfactant.

In some examples, the first surfactant has a mass concentration, in the lysis solution, of 0.1%-10%.

In an example, the first surfactant is an ionic surfactant.

Optionally, the first surfactant is at least one of benzalkonium bromide, sodium dodecyl sulfate, or cetyltrimethylammonium bromide.

In an example, the lysis solution further comprises a protein denaturation agent.

Optionally, the protein denaturation agent is at least one of guanidinium isothiocyanate, guanidinium hydrochloride, a heavy metal salt, or urea.

In some examples, the lysis solution further comprises a protease.

In an example, the protease is proteinase K.

In some examples, the reagent kit further comprises a first washing solution.

In an example, the first washing solution comprises a second surfactant.

In some examples, the second surfactant has a mass concentration, in the first washing solution, of 0.1%-6%.

In an example, the second surfactant is at least one of benzalkonium bromide, polyethylene glycol octyl phenyl ether, sodium dodecyl sulfate, or cetyltrimethylammonium bromide.

In some examples, the reagent kit further comprises a second washing solution.

In an example, the second washing solution comprises ethanol.

In some examples, the reagent kit further comprises a pretreatment reagent.

In an example, the pretreatment reagent is used at a concentration of 1-3 mg/mL.

In an example, the pretreatment reagent comprises lysozyme.

In an example, the pretreatment reagent further comprises a metal ion chelating agent.

Optionally, the metal ion chelating agent is at least one of ethylenediaminetetraacetic acid, nitrilotriacetic acid, or diethylenetriaminepentaacetic acid.

In an example, the reagent kit further comprises a precipitant.

In an example, the precipitant is isopropanol.

In some examples, the nucleic acid sample is a sputum sample, a saliva sample, or a pus sample.

In some other examples, the nucleic acid sample comprises a microbial cell nucleic acid.

In some other examples, the nucleic acid sample comprises catalase.

In another aspect, the present application provides a nucleic acid extraction method, comprising step I: mixing the hydrogen peroxide solution in the above reagent kit with a nucleic acid sample to extract the nucleic acid in the nucleic acid sample.

In some examples, the hydrogen peroxide solution is mixed with the sample for a duration of 0.1-10 min.

In some examples, the method further comprises step II: mixing the pretreatment reagent with the sample.

In some examples, the method further comprises step III: mixing the lysis solution with the sample processed in step II.

In some examples, the method further comprises step IV: mixing the precipitant with the sample processed in step III.

In some examples, the method further comprises step V: mixing the first washing solution with the sample processed in step IV.

In some examples, the method further comprises step VI: mixing the second washing solution with the sample processed in step V.

In the nucleic acid extraction method of the present application, a hydrogen peroxide solution is used to liquefy samples, and the method is capable of liquefying the samples in a shorter duration and a milder manner, reducing nucleic acid loss, and improving the nucleic acid extraction efficiency; the modified lysis solution is capable of better lysing the microbial cells in the samples as well as isolating proteins and nucleic acids and further reducing nucleic acid loss; the modified first washing solution is capable of better removing impurities and improving the purity of the extracted nucleic acids.

### BRIEF DESCRIPTION OF THE DRAWINGS

The above and/or additional aspects and advantages of the embodiments of the present application will be apparent and easily comprehensible from the description of the embodiments with reference to the following drawings, in which:
FIG. 1 shows the results of agarose gel electrophoresis of nucleic acid sample 1 and nucleic acid sample 2 in Example 1;
FIG. 2 shows the result of agarose gel electrophoresis of the bacterial nucleic acids (gDNA) extracted from each group in Example 2.

### DETAILED DESCRIPTION

Embodiments of the present application are described in detail below. The present application may be embodied in many different forms and should not be construed as limited to the examples described herein; these embodiments described below with reference to the accompanying drawings are illustrative and are merely intended to explain the present application, but should not be construed as limiting the present application.

Unless otherwise defined, all technical and scientific terms used herein have the same meaning as commonly understood by those skilled in the art. As used herein, the term "and/or" includes any and all combinations of one or more of the associated listed items.

Currently, viscous samples such as sputum samples, saliva samples, and pus samples need a liquefaction pretreatment prior to nucleic acid extraction, which usually is performed using the sodium hydroxide method that is mainly based on denaturing the proteins in the samples and then dissolving in an alkali liquor using a sodium hydroxide solution with a specific concentration as the principal component to achieve the purpose of sputum liquefaction. However, the method involves the strong alkaline environment provided by the sodium hydroxide solution and is prone to give rise to nucleic acid loss and a relatively low nucleic acid extraction efficiency; in the case that the extraction samples are sputum samples with a relatively low content of *tubercle bacillus,* failure in extracting the *tubercle bacillus* DNA is more than possible to happen using the method.

In order to reduce nucleic acid loss and improve the nucleic acid extraction efficiency during the procedure of nucleic acid extraction from viscous samples, especially sputum samples, the inventors attempt to find a novel method capable of liquefying a sample having the above properties. Through multiple times of experiments, the inventors found that the sample with the properties of high viscosity, high mucin content, difficulties in liquefaction, easiness in agglomeration, etc., can be liquefied using a hydrogen peroxide solution. In addition, the inventors further investigated the effect of the duration of processing the sample with the hydrogen peroxide solution and the concentration for use on nucleic acid extraction and obtained a relatively preferred processing duration and concentration for use. During experiments, the inventors found that liquefying the sample, especially the sputum sample using the hydrogen peroxide solution in a suitable processing duration and concentration for use is capable of reducing nucleic acid loss and improving nucleic acid yield, compared with the sodium hydroxide method.

In addition to the modification of the liquefaction method, the inventors further found that benzalkonium bromide, when serving as one of the ingredients in the lysis solution, is capable of better lysing the sample cells, and when used as a washing solution ingredient for removing impurities such as proteins and the like, is capable of better removing impurities in the procedure of nucleic acid extraction, thereby helping to improve the yield and purity in nucleic acid extraction. Based on the above findings, the inventors made a series of modifications to the nucleic acid extraction method for the sample with the properties of high viscosity, high mucin content, difficulties in liquefaction, easiness in agglomeration, etc., and optimized the nucleic acid extraction method on the whole to improve the nucleic acid yield.

Thus, in one aspect, according to an embodiment of the present application, provided is a reagent kit used in nucleic acid sample extraction, comprising a hydrogen peroxide solution. Nucleic acid extraction from nucleic acid samples using the hydrogen peroxide solution is capable of releasing the nucleic acids from the samples under a mild condition, reducing the destruction of nucleic acid substances, and improving the yield of nucleic acid extraction, the total amount of the extracted microbial cell nucleic acids from a sample.

For viscous samples, especially nucleic acid samples having viscosity indices not less than 20 times larger than that of water under the same condition, microbial cells included therein are wrapped by substances such as mucus or mucin and are difficult to release. After the hydrogen peroxide solution is added, substances in the sample react with hydrogen peroxide to generate gas, and generated bubbles can destroy mucus or block-shaped samples, causing the samples to be completely liquefied, so as to release the microbial cell wrapped by the substances such as mucus or mucin, so that subsequent nucleic acid extraction steps can be smoothly carried out. In addition, the reaction with hydrogen peroxide solution is relatively mild and substantially brings no destruction to cell integrity. This method is capable of reducing nucleic acid loss and improving nucleic acid yield, compared with those using substances such as strong alkali and the like for liquefaction. In the reaction between the substances in the sample and hydrogen peroxide, the gas may be generated from the substances in the samples caused by the hydrogen peroxide or from the decomposition of the hydrogen peroxide caused by the substances in the sample.

In an example, the hydrogen peroxide solution has a molarity of 0.1-2 mol/L. Preferably, in another example, the hydrogen peroxide solution has a molarity of 0.3-1 mol/L. Through multiple times of experiments, the preferred hydrogen peroxide solution concentration for use is determined, and the determination of the concentration for use is based on the sample type and the specific experimental condition.

In the above examples, nucleic acid loss is reduced and the yield of nucleic acid extraction is improved by processing the sample with the hydrogen peroxide solution with a suitable concentration.

In a specific example, the reagent kit further comprises a lysis solution. The lysis solution is used to free the nucleic acids in the sample into the lysis system.

In an example, the lysis solution comprises a first surfactant. In some examples, the first surfactant has a mass concentration, in the lysis solution, of 0.1%-10%.

Preferably, the first surfactant is an ionic surfactant. Preferably, the first surfactant is at least one of benzalkonium bromide, sodium dodecyl sulfate, or cetyltrimethylammonium bromide. The first surfactant may also be a non-ionic surfactant such as ethylphenyl poly(ethylene glycol), polyethylene glycol octyl phenyl ether, or polysorbate-20, and the like. In a specific example, the first surfactant is benzalkonium bromide, which is capable of dissolving lipids and proteins on the cell membrane to destruct the cell membrane and depolymerizing nucleoproteins in the cell to achieve a better lysis effect, thus releasing nucleic acids in the cell more thoroughly and thereby helping to improve the yield of nucleic acid extraction.

In an example, the lysis solution further comprises a protein denaturation agent. The protein denaturation agent is used in rapidly lysing the cell membrane, which is capable of rapidly dissolving proteins, resulting in the fracture of cellular structures, and rapid separation of nucleoproteins from nucleic acids due to the destruction of their secondary structures. Preferably, the protein denaturation agent is at least one of guanidinium isothiocyanate, guanidinium hydrochloride, a heavy metal salt, or urea. Preferably, the protein denaturation agent has a molarity of 4-8 mol/L.

In some examples, the lysis solution further comprises a protease. The protease is used in digesting proteins to small fragments, promoting the separation of nucleic acids from proteins, and meanwhile facilitating obtaining a nucleic acid with higher purity. Preferably, the protease is proteinase K.

The lysis solution in the above examples may comprise multiple types of reagents, and the lysis effect may be achieved by means of one type of reagent, or by means of the combined effect of multiple types of reagents. The composition of the lysis solution may be selected according to different types of nucleic acid extraction samples and different nucleic acid extraction requirements.

In a specific example, the reagent kit may further comprise a precipitant. The precipitant is used in precipitating the nucleic acids obtained by the lysis. Preferably, the precipitant is an organic alcohol. The organic alcohol may be isopropanol, etc., which has high hydrophobicity and has a relatively good effect of precipitating nucleic acid.

In a specific example, the reagent kit further comprises a first washing solution. The first washing solution is used in washing and removing proteins from the nucleic acids obtained by precipitating. In an example, the first washing solution comprises a second surfactant. Preferably, the second surfactant has a mass concentration, in the first washing solution, of 0.1%-6%. Preferably, the second surfactant is at least one of benzalkonium bromide, polyethylene glycol octyl phenyl ether, sodium dodecyl sulfate, or cetyltrimethylammonium bromide. In an example, the second surfactant is benzalkonium bromide, which is capable of improving the purity of the extracted nucleic acid on the premise of ensuring the integrity of the extracted nucleic acid.

In a specific example, the reagent kit further comprises a second washing solution. The second washing solution is used in washing and removing salt ions from the nucleic acids obtained by precipitating. In an example, the second washing solution comprises an organic alcohol. The organic alcohol may be a relatively hydrophilic alcohol, such as methanol, ethanol, propanol, or butanol, in which ethanol has a relatively high hydrophilicity and is capable of removing a portion of salt ions. In an example, the second washing solution further comprises a non-ionic surfactant such as polysorbate-20, polyethylene glycol octyl phenyl ether, and the like. The second washing solution contains a non-ionic surfactant and is capable of further washing and removing proteins from the nucleic acids obtained by precipitating. In another example, the second washing solution further comprises Tris-HCl buffer solution and is capable of washing and removing residual reagents from the previous procedure.

In a specific example, the reagent kit further comprises an eluent. The eluent is used in eluting the nucleic acids from an adsorption matrix, such as magnetic beads, silica matrix membranes, or chromatography columns. In an example, the eluent comprises a Tris-HCl buffer solution.

In a specific example, the reagent kit further comprises a pretreatment reagent. The pretreatment reagent is used in pretreating the liquefied sample to destruct the cell walls of the microbial cells in the sample, facilitating the subsequent sample processing with the lysis solution. Preferably, the pretreatment reagent is used at a concentration of 1-10 mg/mL. Preferably, the pretreatment reagent comprises lysozyme. The lysozyme is capable of lysing bacteria by breaking the β-1,4 glycosidic bond between N-acetylmuramic acid and N-acetylglucosamine in the cell wall, breaking down the cell wall insoluble mucopolysaccharide into soluble glycopeptides, and resulting in cell wall destruction and content leakage. In some examples, the pretreatment reagent further comprises a metal ion chelating agent.

Preferably, the metal ion chelating agent is at least one of ethylenediaminetetraacetic acid, nitrilotriacetic acid, or diethylenetriaminepentaacetic acid.

In a specific example, the nucleic acid sample is a sputum sample, a saliva sample, or a pus sample. The sputum sample has the properties of high viscosity, high mucin content, difficulties in liquefaction, easiness in agglomeration, and the like, and liquefaction processing is needed for the subsequent nucleic acid extraction procedures. Saliva samples indicating a possible occurrence of pathological change in the body also have the properties of high viscosity, high mucin content, and the like, and some pus samples also have relatively high viscosity. Liquefaction processing of these two types of samples is also needed for the subsequent nucleic acid extraction procedures.

In some examples, the nucleic acid sample comprises a microbial cell nucleic acid. The nucleic acid sample may contain some microbial cells, some of which are possibly pathological, such as *mycobacterium tuberculosis, staphylococcus aureus,* and the like. Because some nucleic acid samples have high viscosity, high mucin content, difficulties in liquefaction, easiness in agglomeration, and the like, these microbial cells are wrapped by substances such as mucus or mucin and are difficult to be released. In this case, performing nucleic acid extraction on the samples directly using the lysis solution will have a very low efficiency of microbial cell nucleic acid extraction. Therefore, liquefaction processing is generally performed on such nucleic acid samples to release the microbial cells wrapped in the samples, then the subsequent nucleic acid extraction procedures are performed.

In some examples, the nucleic acid sample comprises catalase. There may be three possible cases where the sample contains catalase: the first case being that the catalase is contained in the samples while collecting the samples; the second case being that the catalase is generated by the microbial cells in the sample prior to sample collection or after sample collection (the inventors summarized, with reference to literature, that most aerobic bacteria, e.g., *mycobacterium tuberculosis,* some facultative anaerobic bacteria, e.g., *staphylococcus aureus,* and a small part of anaerobic bacteria, e.g., *methanosarcina barkeri* are all capable of generating catalase and some of which may be the microbial cells contained in the biological samples such as sputum, saliva, and the like); and the third case being that the catalase contained in the samples is from the samples themselves and the microbial cells in the samples. For samples containing catalase and having high viscosity and easiness in agglomeration, such as sputum samples, saliva samples, and the like, liquefaction processing may be performed on such samples using a hydrogen peroxide solution. The microbial cells wrapped by substances such as mucus or mucin and the like can be released to facilitate the subsequent nucleic acid extraction procedures by breaking the mucus-like samples and block-like samples to fully liquefy them with the gas bubbles resulting from the gas generated using the catalase existing in the samples to decompose the hydrogen peroxide solution. In addition, the reaction between hydrogen peroxide solution and catalase is relatively mild and substantially brings no destruction to cell integrity. This method is capable of reducing nucleic acid loss and improving nucleic acid yield, compared with those using substances such as strong alkali and the like for liquefaction.

In another aspect, according to an embodiment of the present application, provided is a nucleic acid extraction method, comprising step I: mixing the hydrogen peroxide solution in the above reagent kit with a nucleic acid sample. After hydrogen peroxide solution addition, the microbial cells wrapped by substances such as mucus or mucin and the like can be released to facilitate the subsequent nucleic acid extraction procedures by breaking the viscous sample or even the block-forming sample to fully liquefy them with the gas bubbles resulting from the gas generated from the reactions between some substances in the sample and hydrogen peroxide. In addition, the reaction with hydrogen peroxide solution is relatively mild and substantially brings no destruction to cell integrity. This method is capable of reducing nucleic acid loss and improving nucleic acid yield, compared with those using substances such as strong alkali and the like for liquefaction. In the reaction between the substances in the sample and hydrogen peroxide, the gas may be generated from the substances in the samples caused by the hydrogen peroxide or from the decomposition of the hydrogen peroxide caused by the substances in the sample. In some examples, the sample is processed with hydrogen peroxide solution for a duration of 0.1-10 min. The inventors found that the full liquefaction of the sample needs a processing duration of 0.1-10 min using the hydrogen peroxide in a certain range of concentrations to process the sample, but needs a duration of 30 min using the sodium hydroxide method. Compared with the sodium hydroxide solution, the hydrogen peroxide solution is capable of fully liquefying the sample in a shorter duration. The shorter liquefaction duration shortens the time consumption in nucleic acid extraction and improves the efficiency of nucleic acid extraction. Preferably, the sample is processed with the hydrogen peroxide solution for a duration of 0.5-3 min, and the sample can be fully liquefied by processing with the hydrogen peroxide in a suitable concentration for 0.5-3 min.

In some examples, the method further comprises step II: mixing the above pretreatment reagent with the sample. The pretreatment reagent is used in pretreating the liquefied sample to destruct the cell walls of the microbial cells in the sample, facilitating the subsequent sample processing with the lysis solution. Preferably, the pretreatment reagent is used at a concentration of 1-10 mg/mL. Preferably, the pretreatment reagent comprises lysozyme. The lysozyme is capable of lysing bacteria by breaking the β-1,4 glycosidic bond between N-acetylmuramic acid and N-acetylglucosamine in the cell wall, breaking down the cell wall insoluble mucopolysaccharide into soluble glycopeptides, and resulting in cell wall destruction and content leakage. In some examples, the pretreatment reagent further comprises a metal ion chelating agent. Preferably, the metal ion chelating agent is at least one of ethylenediaminetetraacetic acid, nitrilotriacetic acid, or diethylenetriaminepentaacetic acid. In an example, the lysis solution is mixed with the sample before mixing the pretreatment reagent with the sample to destruct the biomembranes in the sample that may be incompletely liquefied, thereby promoting the destruction effect of the pretreatment reagent on the cell walls of the microbial cells in the sample.

In some examples, the method further comprises step III: mixing the lysis solution with the sample processed in step II, wherein the lysis solution is used to free the nucleic acids in the sample into the lysis system, and specifically, the lysis solution is capable of destructing the cell membranes of the microbial cells or cells contained in the sample and releasing the nucleic acids from these cells. After mixing the lysis solution with the sample processed in step II, the nucleic acids contained in the sample are released for performing the subsequent nucleic acid precipitation procedure.

In an example, the lysis solution comprises a first surfactant. In some examples, the first surfactant has a mass concentration, in the lysis solution, of 0.1%-10%. Preferably, the first surfactant is an ionic surfactant. Preferably, the first surfactant is at least one of benzalkonium bromide, sodium dodecyl sulfate, or cetyltrimethylammonium bromide. The first surfactant may also be a non-ionic surfactant such as ethylphenyl poly(ethylene glycol), polyethylene glycol octyl phenyl ether, or polysorbate-20, and the like. In a specific example, the first surfactant is benzalkonium bromide, which is capable of dissolving lipids and proteins on the cell membrane to destruct the cell membrane and depolymerizing nucleoproteins in the cell to achieve a better lysis effect, thus releasing nucleic acids in the cell more thoroughly and thereby helping to improve the yield of nucleic acid extraction.

In an example, the lysis solution further comprises a protein denaturation agent. The protein denaturation agent is used in rapidly lysing the cell membrane, which is capable of rapidly dissolving proteins, resulting in the fracture of cellular structures, and rapid separation of nucleoproteins from nucleic acids due to the destruction of their secondary structures. Preferably, the protein denaturation agent is at least one of guanidinium isothiocyanate, guanidinium hydrochloride, a heavy metal salt, or urea. Preferably, the protein denaturation agent has a molarity of 4-8 mol/L.

In some examples, the lysis solution further comprises a protease. The protease is used in digesting proteins to small fragments, promoting the separation of nucleic acids from proteins, and meanwhile facilitating obtaining a nucleic acid with higher purity. Preferably, the protease is proteinase K.

The lysis solution in the above examples may comprise multiple types of reagents, and the lysis effect may be achieved by means of one type of reagent, or by means of the combined effect of multiple types of reagents. The composition of the lysis solution may be selected according to different types of nucleic acid extraction samples and different nucleic acid extraction requirements.

In some examples, the method further comprises step IV: mixing the precipitant with the sample processed in step III. In this step, the nucleic acids released from the sample in step III are precipitated using the above precipitant. Preferably, the precipitant is an organic alcohol. The organic alcohol may be isopropanol, etc., which has high hydrophobicity and has a relatively good effect of precipitating nucleic acid.

In some examples, the method further comprises step V: mixing the first washing solution with the sample processed in step IV, wherein the first washing solution is used for washing and removing proteins from the nucleic acids obtained by precipitating. In an example, the first washing solution comprises a second surfactant. Preferably, the second surfactant has a mass concentration, in the first washing solution, of 0.1%-6%. Preferably, the second surfactant is at least one of benzalkonium bromide, polyethylene glycol octyl phenyl ether, sodium dodecyl sulfate, or cetyltrimethylammonium bromide. In an example, the second surfactant is benzalkonium bromide, which is capable of improving the purity of the extracted nucleic acid on the premise of ensuring the integrity of the extracted nucleic acid.

In some examples, the method further comprises step VI: mixing the second washing solution with the sample processed in step V for washing. The second washing solution is used in washing and removing salt ions from the nucleic acids obtained by precipitating. In an example, the second washing solution comprises an organic alcohol. The organic alcohol may be a relatively hydrophilic alcohol, such as methanol, ethanol, propanol, or butanol, in which ethanol has a relatively high hydrophilicity and is capable of removing a portion of salt ions. In an example, the second washing solution further comprises a non-ionic surfactant such as polysorbate-20, polyethylene glycol octyl phenyl ether, and the like. The second washing solution contains a non-ionic surfactant and is capable of further washing and removing proteins from the nucleic acids obtained by precipitating. In another example, the second washing solution further comprises Tris-HCl buffer solution and is capable of washing and removing residual reagents from the previous procedure. In an example, the nucleic acids processed in step V may be washed with the second washing solution containing various different ingredients multiple times to better remove impurities. For example, the sample processed in step V may be washed once with the second washing solution containing an organic alcohol, e.g., ethanol, a non-ionic surfactant, e.g., polysorbate-20, as well as Tris-HCl buffer solution and washed again with the second washing solution containing an organic alcohol, e.g., ethanol as well as Tris-HCl buffer solution. The composition of the second washing solution and the order and the number of the washing can be selected according to the requirements of the experiment.

In the nucleic acid extraction method of the present application, a hydrogen peroxide solution is used to liquefy nucleic acid samples, and the method is capable of liquefying the samples in a shorter duration and a milder manner, reducing nucleic acid loss, and improving the nucleic acid extraction efficiency; the modified lysis solution is capable of better lysing the microbial cells in the samples as well as isolating proteins and nucleic acids and further reducing nucleic acid loss; the modified first washing solution is capable of better removing impurities and improving the purity of the extracted nucleic acids, and the nucleic acid yield, the nucleic acid extraction efficiency and the extracted nucleic acid purity are improved by the interplay of the multiple procedures.

The present application is described in detail below by means of specific examples, and it will be appreciated that the examples are exemplary only. The materials, reagents, sequences, and the like mentioned in the examples can be prepared or synthesized or commercially available, unless otherwise specified.

### Example 1

This example relates to the specific procedures and the related results of the sputum sample liquefaction.
1. Sputum liquefaction
1.1 LP bacterial solution of 10⁸ CFU was added to each of sputum sample 1, sputum sample 2, sputum sample 3, sputum sample 4, and sputum sample 5 that were isovolumetric. The mixture was homogeneously mixed;
1.2 1 mol/L NaOH solution, 0.1 mol/L H₂O₂ solution, 0.3 mol/L H₂O₂ solution, 1 mol/L H₂O₂ solution, and 2 mol/L H₂O₂ solution were added isovolumetrically to sputum sample 1, sputum sample 2, sputum sample 3, sputum sample 4, and sputum sample 5 respectively and the samples were observed. Full sample liquefaction was manifested by the samples having no noticeable solid impurities and being not viscous. According to the observation, sputum sample 1 with NaOH solution addition liquefied at 30 min; sputum sample 2 with 0.1 mol/L H₂O₂ solution addition liquefied at 10 min; sputum sample 3 with 0.3 mol/L H₂O₂ solution addition liquefied at 1 min; sputum sample 4 with 1 mol/L H₂O₂ solution addition liquefied at 0.5 min; and sputum sample 5 with 2 mol/L H₂O₂ solution addition liquefied at 0.5 min. In this example, sputum sample 1 and sputum sample 3 were separately centrifuged and the supernatants were discarded to give liquefied sample 1 and liquefied sample 2.

2. Sample pretreatment
2.1 500 µL of 1× PBS was added into each of liquefied sample 1 and liquefied sample 2 and homogeneously mixed by shaking, and the mixture was centrifuged at 10000× g for 5 min. The supernatant was discarded.
2.2 110 µL of lysis solution was added to each of liquefied sample 1 and liquefied sample 2 processed in 2.1. The mixture was homogeneously mixed, and 70 µL of pretreatment reagent containing lysozyme with a mass concentration of 10 mg/mL was added. The mixture was incubated at 37 °C for 30 min.

3. Nucleic acid extraction
3.1 300 µL of lysis solution containing benzalkonium bromide was added to each of liquefied sample 1 and liquefied sample 2 processed in 2.2 The mixture was homogeneously mixed, and 20 µL of proteinase K was added. The mixture was left to stand at 70 °C for 15 min.
3.2 The mixture was left to stand at room temperature for 3 min. While the temperature of the tubes decreased to room temperature, 350 µL of isopropanol was added to each tube, and the mixture was homogeneously mixed.
3.3 40 µL of silica-coated magnetic beads were added to each tube, and the mixture was homogeneously mixed and left to stand at room temperature for 5 min.
3.4 The tubes were placed on a magnetic rack and left to stand for 3 min; the supernatant was discarded.
3.5 700 µL of the first washing solution containing benzalkonium bromide was added to each tube, and the mixture was homogeneously mixed by thoroughly shaking. The tubes were placed on a magnetic rack and left to stand for 2 min; the supernatant was discarded.
3.6 700 µL of the second washing solution A was added to each tube, and the mixture was homogeneously mixed by thoroughly shaking. The tubes were placed on a magnetic rack and left to stand for 2 min; the supernatant was discarded.
3.7 700 µL of the second washing solution B was added to each tube, and the mixture was homogeneously mixed by thoroughly shaking. The tubes were placed on a magnetic rack and left to stand for 2 min; the supernatant was discarded.
3.8 The EP tube covers were open, and the mixture was dried at room temperature for 5 min avoiding overdrying of the magnetic beads.
3.9 100 µL of eluent was added to each tube, and the mixture was homogeneously mixed. The tubes were placed on a magnetic rack; the supernatants, as nucleic acid sample 1 and nucleic acid sample 2, were aspirated.

4. Results of nucleic acid extraction assay
4.1 Nucleic acid sample 1 and nucleic acid sample 2 obtained from nucleic acid extraction were subjected to Qubit assay (for nucleic acid concentration), Nanodrop assay (for nucleic acid concentration), OD260/280 ratio (indication value of nucleic acid purity) assay, and OD260/230 ratio (indication value of nucleic acid purity) assay. The results are shown in Table 1:

**Table 1**

| No. | Assay sample | Nucleic acid concentration (ng/ µL) by Qubit assay | Nucleic acid concentration (ng/µL) by Nanodrop assay | OD260/28 0 value | OD260/230 value |
|---|---|---|---|---|---|
| 1 | Nucleic acid sample 1 (NaOH) | 8.22 | 16.9 | 1.88 | 1.27 |
| 2 | Nucleic acid sample 2 (H₂O₂) | 48.4 | 103.3 | 1.87 | 1.61 |
| 3 | Pure LP bacterial solution 1 | 65.4 | 214 | 2.06 | 2.44 |
| 4 | Pure LP bacterial solution 2 | 50.4 | 156.1 | 1.97 | 2.03 |

| | | | | | |
|---|---|---|---|---|---|
| Note: pure LP bacterial solution 1 and pure LP bacterial solution 2 are unprocessed bacterial solutions with identical components and contents. | | | | | |

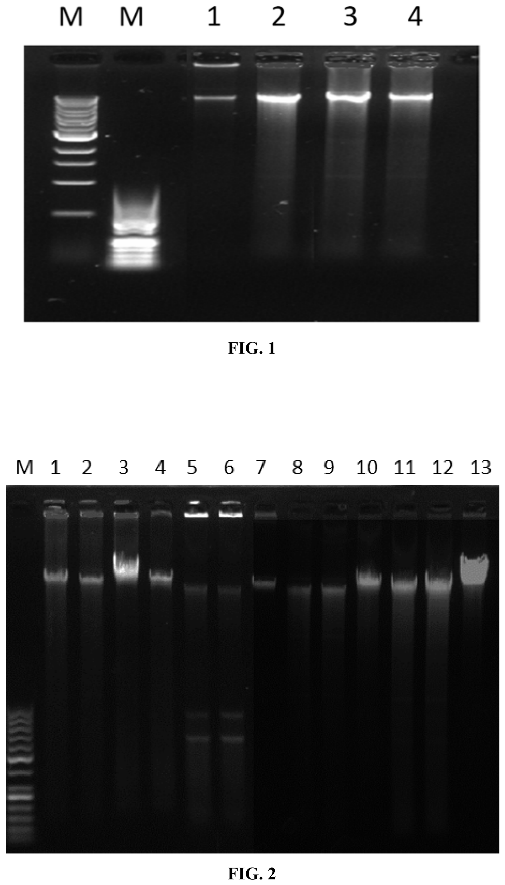

4.2 Nucleic acid sample 1 and nucleic acid sample 2 obtained from nucleic acid extraction were subjected to agarose gel electrophoresis, and the obtained results are shown in FIG. 1.

As can be seen from the results of 4.1 and 4.2, the time consumption using the sodium hydroxide solution to liquefy the sputum sample is longer than that using the hydrogen peroxide solutions. Furthermore, nucleic acids extracted from those samples liquefied using the hydrogen peroxide solutions had concentrations far higher than that extracted from the sample liquefied using the sodium hydroxide solution, as determined by Qubit assay (for nucleic acid concentration) and Nanodrop assay (for nucleic acid concentration). In terms of purity, the sample liquefied using the sodium hydroxide solution and the samples liquefied using the hydrogen peroxide solutions had OD260/280 ratios suggesting substantially identical purities of nucleic acids extracted from them and OD260/230 ratios suggesting higher purities of nucleic acids extracted from the latter. The above results demonstrate that sputum sample liquefaction using the hydrogen peroxide solutions had the advantages of reduced nucleic acid loss, high yield of nucleic acid extraction, shorter liquefaction duration, improved nucleic acid extraction efficiency, as well as prime purity. As can be seen from the results of agarose gel electrophoresis, both nucleic acid sample 1 and nucleic acid sample 2 had only one apparent and relatively intact electrophoresis band with a slightly smearing tail and had no additional smearing phenomenon and small fragment bands, suggesting relatively good electrophoresis DNA integrity. The nucleic acid extraction method of the present application had good nucleic acid integrity in extraction.

### Example 2

This example compares the results of nucleic acid extraction using the reagent kit of the present application and other commercially available reagent kits (Tiangen^{™}, Gene Optimal^{™}, and Vazyme^{™}) from gram-positive bacteria and gram-negative bacteria in sputum samples. In this example, *Lactobacillus plantarum* (LP, for short) was used as a representative of gram-positive bacteria, and *Escherichia coli* (EC, for short) was used as a representative of gram-negative bacteria.

In this example, grouping was performed according to the types of reagent kits and the types of strains, and the specific grouping is shown in Table 2:

**Table 2**

| Group | Reagent kit | Type of strain |
|---|---|---|
| 1 | The reagent kit of the present application | LP-1 |
| 2 | | LP-2 |
| 3 | | EC-W |
| 4 | | EC-N |
| 5 | Tiangen^{™} | LP-1 |
| 6 | | LP-2 |
| 7 | | EC |
| 8 | Gene Optimal^{™} | LP-1 |
| 9 | | LP-2 |
| 10 | | EC |
| 11 | Vazyme^{™} | LP-1 |
| 12 | | LP-2 |
| 13 | | EC |

| | | |
|---|---|---|
| Note: W and N denote magnetic beads from different manufacturers. | | |

The nucleic acid extraction was completed according to the procedures of each reagent kit, and the bacterial nucleic acid (gDNA) extracted from each group was subjected to agarose gel electrophoresis assay to confirm DNA integrity (the results are shown in FIG. 2) and Qubit assay (for nucleic acid concentration), Nanodrop assay (for nucleic acid concentration), OD260/280 ratio (indication value of nucleic acid purity) assay, and OD260/230 ratio (indication value of nucleic acid purity) assay. The results are shown in Table 3.

**Table 3**

| Group | Reagent kit | Type of strain | Concentration (ng/µL) by Qubit assay | Concentration (ng/µL) by Nanodrop assay | OD260/280 | OD 260/230 |
|---|---|---|---|---|---|---|
| 1 | The reagent kit of the present application | LP-1 | 214 | 226 | 1.84 | 1.80 |
| 2 | | LP-2 | 216 | 244 | 1.82 | 1.63 |
| 3 | | EC-W | 125 | 148 | 1.82 | 1.36 |
| 4 | | EC-N | 171 | 175 | 1.99 | 1.44 |
| 5 | Tiangen^{™} | LP-1 | 13.1 | 205 | 1.49 | 0.74 |
| 6 | | LP-2 | 15.7 | 181.1 | 1.52 | 0.77 |
| 7 | | EC | 14.4 | 43.8 | 1.63 | 0.81 |
| 8 | Gene Optimal^{™} | LP-1 | 34.6 | 48.1 | 1.93 | 2.12 |
| 9 | | LP-2 | 23.4 | 37.1 | 1.9 | 2.03 |
| 10 | | EC | 40 | 60.9 | 1.92 | 1.81 |
| 11 | Vazyme^{™} | LP-1 | 50 | 90.1 | 1.9 | 0.73 |
| 12 | | LP-2 | 30 | 108.7 | 1.9 | 0.74 |
| 13 | | EC | 70 | 149.8 | 1.88 | 0.63 |

As can be seen from the results for agarose gel electrophoresis (see FIG. 2, where M is 50 bp Marker) of the bacterial gDNAs extracted by the four reagent kits. Lanes 5, 6, and 7 in FIG. 2 show apparent fluorescence in the electrophoresis loading wells, suggesting that the LP nucleic acids extracted by Tiangen^{™} reagent kit contain relatively high protein residuals, which bind to nucleic acids with relatively large fragments and render them incapable of leaving the loading wells, thereby generating fluorescence in the loading wells; furthermore, multiple relatively dim bands in the lanes suggest that the extracted nucleic acids contain small fragment DNAs, indicating cleavage of the extracted LP gDNAs and high protein contents. Lanes 8-13 in FIG. 2 show barely noticeable fluorescence in the loading wells, suggesting that the nucleic acids extracted by Gene Optimal^{™} and Vazyme^{™} reagent kits contain relatively fewer protein residuals and these two reagent kits are relatively advantageous in removing proteins. Lanes 1-4 in FIG. 2 show a relatively apparent smearing phenomenon but only one band and no other small fragment DNAs in each lane, suggesting that the nucleic acids extracted by the reagent kit of the present application have better nucleic acid integrity; no noticeable fluorescence in the loading wells indicates less protein impurities and higher purity of the extracted nucleic acids. The reagent kit of the present application is capable of better removing protein residuals and improving the purity of the extracted nucleic acid on the premise of ensuring nucleic acid integrity, possibly due to the optimization of the first washing solution.

As seen from the results of Qubit assay, Nanodrop assay, OD260/280 ratio assay, and OD260/230 ratio assay in Table 3, the concentrations of the nucleic acids extracted from the gram-positive bacteria and the gram-negative bacteria using the reagent kit of the present application were significantly higher than those using other reagent kits, so was the purity. The reagent kit of the present application optimized and modified the liquefaction processing method, the pretreatment reagent, the lysis solution, the first washing solution, and the second washing solution, etc. Thus the liquefaction process has short time consumption, full liquefaction, and reduced nucleic acid loss; the modified lysis solution possesses a better lysis effect and is capable of further reducing nucleic acid loss; the modified washing solution is capable of better removing impurities such as proteins, salt ions, and the like and improving the purity of the extracted nucleic acid. The combined effect of the modification of multiple types of reagents has improved the concentration and purity of the extracted nucleic acid, as well as improved the efficiency and yield of nucleic acid extraction, as can be seen from the experimental results.

In the description of this specification, the description of the terms "one embodiment", "some embodiments", "schematic embodiments", "examples", "certain examples", "specific examples", "embodiment", or the like, means that the particular features, structures, materials, or characteristics described with reference to the embodiment or example are included in at least one embodiment or example of the present application. In this specification, the schematic description of the above terms does not necessarily refer to the same embodiment or example. Moreover, the particular features, structures, materials, or characteristics described may be combined in any one or more embodiments or examples in any appropriate manner.

Although the embodiments of the present application have been shown and described above, it is to be understood that the above embodiments are exemplary and are not to be construed as limiting the present application, and that those of ordinary skill in the art may make changes, modifications, replacements and variations to such embodiments without departing from the scope of the present application.

## Claims

1. A reagent kit used in nucleic acid sample extraction,
comprising a hydrogen peroxide solution having a molarity of 0.1-2 mol/L.

2. The reagent kit according to claim 1, wherein the hydrogen peroxide solution has a molarity of 0.3-1 mol/L.

3. The reagent kit according to any one of claims 1-2, further comprising a lysis solution.

4. The reagent kit according to claim 3, wherein the lysis solution comprises a first surfactant.

5. The reagent kit according to claim 4, wherein the first surfactant has a mass concentration, in the lysis solution, of 0.1%-10%.

6. The reagent kit according to claim 4 or 5, wherein the first surfactant is an ionic surfactant.

7. The reagent kit according to any one of claims 4-6, wherein the first surfactant is at least one of benzalkonium bromide, sodium dodecyl sulfate, or cetyltrimethylammonium bromide.

8. The reagent kit according to any one of claims 3-7, wherein the lysis solution further comprises a protein denaturation agent.

9. The reagent kit according to claim 8, wherein the protein denaturation agent is at least one of guanidinium isothiocyanate, guanidinium hydrochloride, a heavy metal salt, or urea.

10. The reagent kit according to any one of claims 3-9, wherein the lysis solution further comprises a protease.

11. The reagent kit according to claim 10, wherein the protease is proteinase K.

12. The reagent kit according to any one of claims 1-11, further comprising a first washing solution.

13. The reagent kit according to claim 12, wherein the first washing solution comprises a second surfactant.

14. The reagent kit according to claim 13, wherein the second surfactant has a mass concentration, in the first washing solution, of 0.1%-6%.

15. The reagent kit according to claim 13 or 14, wherein the second surfactant is at least one of benzalkonium bromide, polyethylene glycol octyl phenyl ether, sodium dodecyl sulfate, or cetyltrimethylammonium bromide.

16. The reagent kit according to any one of claims 1-15, wherein the reagent kit further comprises a second washing solution.

17. The reagent kit according to claim 16, wherein the second washing solution comprises an organic alcohol.

18. The reagent kit according to any one of claims 1-17, further comprising a pretreatment reagent.

19. The reagent kit according to claim 18, wherein the pretreatment reagent is used at a concentration of 1-3 mg/mL.

20. The reagent kit according to claim 18 or 19, wherein the pretreatment reagent comprises lysozyme.

21. The reagent kit according to any one of claims 18-20, wherein the pretreatment reagent further comprises a metal ion chelating agent.

22. The reagent kit according to claim 21, wherein the metal ion chelating agent is at least one of ethylenediaminetetraacetic acid, nitrilotriacetic acid, or diethylenetriaminepentaacetic acid.

23. The reagent kit according to any one of claims 1-22, further comprising a precipitant.

24. The reagent kit according to claim 23, wherein the precipitant is an organic alcohol.

25. The reagent kit according to any one of claims 1-24, wherein the nucleic acid sample is a sputum sample, a saliva sample, or a pus sample.

26. The reagent kit according to any one of claims 1-25, wherein the nucleic acid sample comprises a microbial cell nucleic acid.

27. The reagent kit according to any one of claims 1-26, wherein the nucleic acid sample comprises catalase.

28. A nucleic acid extraction method, comprising
step I: mixing the hydrogen peroxide solution in the reagent kit according to any one of claims 1-24 with a nucleic acid sample to extract a nucleic acid in the nucleic acid sample.

29. The method according to claim 28, wherein the hydrogen peroxide solution is mixed with the sample for a duration of 0.1-10 min.

30. The method according to claim 28, further comprising step II: mixing the pretreatment reagent with the sample.

31. The method according to claim 28 or 29, further comprising step III: mixing the lysis solution with the sample processed in step II.

32. The method according to any one of claims 28-30, further comprising step IV: mixing the precipitant with the sample processed in step III.

33. The method according to any one of claims 28-31, further comprising step V: mixing the first washing solution with the sample processed in step IV.

34. The method according to any one of claims 28-32, further comprising step VI: mixing the second washing solution with the sample processed in step V.
